# EUROPEAN PATENT SPECIFICATION

(11) **EP 2 374 871 B1**
(45) Date of publication and mention of the grant of the patent: **06.04.2016**
(21) Application number: 09832886.7
(22) Date of filing: 23.10.2009
(51) Int. Cl.: C12N 5/073, A61K 35/12, C12N 5/074

(54) **PLURIPOTENT STEM CELLS, METHOD FOR PREPARATION THEREOF AND USES THEREOF**
PLURIPOTENTE STAMMZELLEN, VERFAHREN ZU IHRER HERSTELLUNG UND VERWENDUNG
CELLULES SOUCHES PLURIPOTENTES, PROCÉDÉ POUR LA PRÉPARATION DE CELLES-CI ET UTILISATIONS DE CELLES-CI

(30) Priority: 17.12.2008 CN 200810240040
(43) Date of publication of application: 12.10.2011
(73) Proprietor: Beijing Health & Biotech (H&B) Co., Ltd, Beijing 100176 (CN)
(72) Inventor: ZHAO, Baona, Beijing 100176 (CN); HAN, Zhihai, Beijing 100176 (CN)
(74) Representative: Schmid, Wolfgang
(86) International application number: PCT/CN2009/074581
(87) International publication number: WO 2010/069204

(56) References cited:
- WO-A1-2008/144820
- WO-A1-2008/148105
- WO-A2-2005/042730
- US-A1- 2003 161 818
- US-A1- 2008 152 629
- US-A1- 2008 274 087
- LU LU-LU ET AL: "Isolation and characterization of human umbilical cord mesenchymal stem cells with hematopoiesis-supportive function and other potentials", HAEMATOLOGICA, FONDAZIONE FERRATA STORTI, ROME, IT, vol. 91, no. 8, 1 August 2006 (2006-08-01) , pages 1017-1026, XP009110905, ISSN: 0390-6078
- IWAI KAORI ET AL: "Expression and function of transmembrane-4 superfamily (tetraspanin) proteins in osteoclasts: reciprocal roles of Tspan-5 and NET-6 during osteoclastogenesis.", ALLERGOLOGY INTERNATIONAL : OFFICIAL JOURNAL OF THE JAPANESE SOCIETY OF ALLERGOLOGY DEC 2007, vol. 56, no. 4, December 2007 (2007-12), pages 457-463, XP9165948, ISSN: 1323-8930
- ILANCHERAN S ET AL: "Human Fetal Membranes: A Source of Stem Cells for Tissue Regeneration and Repair?", PLACENTA, W.B. SAUNDERS, GB, vol. 30, no. 1, 1 November 2008 (2008-11-01), pages 2-10, XP025814584, ISSN: 0143-4004, DOI: 10.1016/J.PLACENTA.2008.09.009 [retrieved on 2008-11-07]
- MIHU CARMEN MIHAELA ET AL: "Isolation and characterization of stem cells from the placenta and the umbilical cord", ROMANIAN JOURNAL OF MORPHOLOGY AND EMBRYOLOGY = REVUE ROUMAINE DE MORPHOLOGIE ET EMBRYOLOGIE, BUCURE TI : ED. ACAD. ROMÂNE, vol. 49, no. 4, 1 January 2008 (2008-01-01), pages 441-446, XP009165946, ISSN: 1220-0522
- LI, CAIXIA ET AL.: 'Isolation and identification of multipotent stem cells from human umbilical cord.' IMMUNOLOGICAL JOURNAL. vol. 24, no. 5, September 2008, pages 583 - 587, XP008166801

## Description

### Field of the invention

This invention relates to stem cells. More particularly, this invention relates to pluripotent stem cells derived from human umbilical cord or placenta. Moreover, this invention relates to the preparative method of the pluripotent stem cells. Still more, this invention relates to the uses of the pluripotent stem cells.

### Background of the invention

The human body is composed of trillions of cells. A cell is the fundamental structural and functional unit in the body and differentiates from stem cell. Therefore, the stem cells are original cells of all types of organs and tissues in the body and characteristic of self-renewal and multipotent differential capacity. According to normal development process, the stem cells are differentiated from zygote. Zygote initially form primordial embryonic stem cells, which have the potential of develop into a fetal or adult animal. And then primordial embryonic stem cells differentiate into blastocyst, in which there are some cells named inner cell mass or embryonic stem cell. Single embryonic stem cell can not develop into a fetal or adult animal, but is able to differentiate into any cell in the organism. Embryoblast continue to differentiate into all type of functional cell in a fetal, at the same time, retain some stem cells having different potential by the way of asymmetric cell division in the tissues, especially hematopoietic tissue, include placenta and cord blood, bone marrow, peripheral blood, liver and spleen. During the whole development process, the stem cells lose their totipotency, and step by step form pluripotent stem cells, multipotent stem cells and specialized stem cells. All these stem cells play the key roles in the growth and development of human organs and tissues and their repair from damage.

Up to now, specialized stem cells such as hemopoietic stem cells have been used extensively in clinical therapy; meanwhile, researches on embryonic stem cells have made great progress. But pluripotent stem cells are still very strange to researchers except similar to embryonic stem cells in developmental stage and differential potential, displaying some feature of embryonic stem cells, possessing many biological characters of multipotent stem cells and forming no teratoma after injection into nude mice.

In fact, pluripotent stem cells have wide perspective in clinical application and have become a new hot spot in the field of stem cells, because they, as a member of adult stem cells, avoid from the medical ethical problems faced by embryonic stem cells.

### Summary of the invention

The present invention provides a population of pluripotent stem cells derived from human umbilical cord or placenta. These pluripotent stem cells are able to be used as carrier cells of gene therapy and for the treatment of diseases caused by cell damage or cell aging.

In another aspect, the present invention provides a method for preparing a population of human pluripotent stem cells.

In a further aspect, the present invention provides usages of these pluripotent stem cells.

The pluripotent stem cells are characterized as being CD151, OCT4 positive and CD 184 negative. OCT4 is a specific marker of embryonic stem cells. CD 151 is a marker commonly found on some stem cells, epidermic cells, endothelial cells, thrombocytes, dendritic cells and not on lymphocytes, monocytes and granulocytes. CD184 is chemokine receptor 4(CXCR4), which is expressed on lymphocyte, monocyte, dendritic cell, granulocyte, endothelial cell, epidermic cell and CD34⁺ stem cell. In addition, the population of cells of the present invention expresses other main immunological marker of embryonic stem cells, mesenchymal stem cells, neural stem cells and vascular stem cells, include Sox-2, Nestin, CD90, CD29, CD13, CD166, CD105, CD44, CD73, CD49e, GD2 and HLA-I, but lacks expression of CD34, CD31, CD45, CD133, CD106, CD11b, CD271, CD41, CD61, CD42b and HLA-II.

The pluripotent stem cells are also characterized as being able to adhere to tissue culture plastic and having the potential to differentiate into three germ layers: endoderm, mesoderm and ectoderm, which including without limitation blood vessel, nerve, hepar, myocardium, bone, cartilage and adipose tissue. Furthermore, the population of cells of this invention do not form teratoma after injection into animals.

The present invention provides a method of isolating, purifying and culturally expanding of a population of human pluripotent stem cells, comprising
(a) cutting and collecting human umbilical cord and or placenta tissues by aseptic processing; and
(b) mincing collected tissues into fragments and incubating the fragments with protease and then passing through a filter to obtain primary mononuclear cells; and
(c) seeding the mononuclear cells in culture flasks and maintaining in grow medium I or II and expanding cells through passage 4 or above; and collecting the cells then cryopreserving in liquid nitrogen.

Grow medium I consist of 50-70% DMED/F12 and 30-50% MCDB-201, supplemented with 2-10% serum, 10⁻⁸ mol/L dexamethasone, 10-50mg/mL insulin-transferrin-selenium(ITS), 0.1-10 mmol/L glutamine, 1-100 ng/mL human epidermal growth factor(hEGF) and 1-100 ng/mL basic fibroblast growth factor(bFGF).

Grow medium II consist of 50-70% DMED/F12 and 30-50% MCDB-201, supplemented with 0.1-5%(W/V) human serum albumin(HSA), 1-100 µ g/mL linolenic acid, 1-100 µ g/mL linoleic acid, 0.1-5% non-essential amino acid, 10⁻⁸mol/L dexamethasone, 10-50mg/mL insulin-transferrin-selenium(ITS), 0.1-10 mmol/L glutamine, 1-100 ng/mL human epidermal growth factor(hEGF) and 1-100 ng/mL basic fibroblast growth factor(bFGF).

The present invention provides a method of isolating, purifying and culturally expanding of a population of human pluripotent stem cells, comprising
(a) cutting and collecting human umbilical cord and or placenta tissues by aseptic processing; and
(b) mincing collected tissues into fragments and incubating the fragments with protease and then passing through a filter to obtain primary mononuclear cells; and
(c) selecting CD151+, OCT4+ and CD 184- cells from the mononuclear cells through Magnetic Activated Cell Sorting and cryopreserving in liquid nitrogen.

The present invention provides a method of preparing pluripotent stem cells wherein said the protease is collagenase type I or trypsin.

The present invention provides a usage of pluripotent stem cells being carrier cells in gene therapy.

The present invention provides a usage of pluripotent stem cells treating diseases caused by cell damage or cell aging.

The present invention provides a usage of pluripotent stem cells promoting the differentiation of stem cells into lipoblast, osteoblast, chondroblast, myocardial cell, nerve cell, hepatocyte and stimulating the haematogenesis.

The present invention provides a usage of pluripotent stem cells treating diseases of immunologic abnormality.

The present invention provides a usage of pluripotent stem cells treating brain damage.

The present invention provides a usage of pluripotent stem cells treating graft-versus-host disease.

Beneficial effects of the present invention lie in:
1. The population of CD151⁺, CD184⁻, OCT4⁺ pluripotent stem cells obtained according to the method provided by this invention can be used as carrier of gene therapy, which means transfecting special gene into vector cells and transplanting these genetically modified vector cells into body directly so as to treat related diseases.
2. The population of CD151⁺, CD184⁻, OCT4⁺ pluripotent stem cells obtained according to the method provided by this invention can be isolated from one's own or other people's cord or placenta and produce into stem cells formula for experimental study and clinical treatment use.
3. The population of CD151⁺, CD184⁻, OCT4⁺ pluripotent stem cells obtained according to the method provided by this invention can be administered locally or systemically, such as by intravenous administration.

The following figures and examples are provided to further illustrate and describe the present invention; however, the scope of the present invention is not intended to be limited thereby.

### Brief description of the drawings

Fig.1 Surface markers of pluripotent stem cells (passage 6) determined by flow cytometry
Fig.2 Internal markers of pluripotent stem cells determined by flow cytometry
Fig.3 Image of adipogenic differentiation
Fig.4 Image of osteogenic differentiation
Fig.5 Image of chondrogenic differentiation
Fig.6 Electrophoretogram of pluripotent stem cells differentiating into myocardial cell
Fig.7 Electrophoretogram of pluripotent stem cells differentiating into nerve cell
Fig.8 Electrophoretogram of pluripotent stem cells differentiating into hepatocyte
Fig.9 Fluorescence image of pluripotent stem cells 48 hours post-transfection with Ad-GFP
Fig.10 Results of pluripotent stem cells treating rat hemorrhagic brain injured
Fig.11 Chart of pluripotent stem cells supporting the growth of hemopoietic stem cell
Fig.12A Effect of pluripotent stem cells on proliferation of mice spleen cells stimulated by ConA
Fig.12B Effect of pluripotent stem cells on IFN-γ secretion of mice spleen cells stimulated by ConA
Fig.12C Effect of pluripotent stem cells on proliferation of human PBMC stimulated by PHA
Fig.12D Effect of pluripotent stem cells on IFN-γ secretion of human PBMC stimulated by PHA

### Detailed description of the embodiments

The following are descriptions of culture medium, terms, and techniques which may be involved in the present invention:
*Culture medium and nutritional ingredient for stem cells:*
   DMEM/F12 (Invitrogen)
   MCDB-201 (Sigma)
   ITS (insulin transferrin selenium): mixture of insulin, transferring and sodium selenate, from Sigma
   Non-essential amino acid: mixed liquor of non-essential amino acid, from Sigma
*Terms:*
   Positive expression: greater than or equal to 70%
   Negative expression: less than or equal to 5%
*Techniques:*
   Techniques employed in the present invention include flow cytometry, Magnetic Activated Cell Sorting , RT-PCR, cell culture and so on are refer to following book or monograph:
      Theory and Transplantation of Hemopoietic Stem Cells, chiefly edited by Zhongchao Han, Henan Science and Technology Press, 2000
      Isolation and characterization of human umbilical cord mesenchymal stem cells with hematopoiesis-supportive function and other potentials. Haematologica, 91(8):1017-26,2006

Except defined especially, the terms in the present invention means common definition know by the technicians of the field and the techniques means common method of the field.

*Reparation of cell culture medium:*
Medium A:
   5L DMEM/F12; 5L MCDB-201; 2%FBS; 10-8 mol/L dexamethasone; 10mg/mL ITS; 0.1 mmol/L glutamine; 1ng/mL hEGF; 1 ng/mL bFGF
Medium B:
   7L MEM/F12; 3L MCDB-201; 10% FBS; 10-8 mol/L dexamethasone;
   50mg/mL ITS; 10 mmol/L glutamine; 100 ng/mL hEGF; 100 ng/mL bFGF.
Medium C:
   6L DMEM/F12; 4L MCDB-201; 6%FBS; 10-8 mol/L dexamethasone; 30mg/mL ITS; 5 mmol/L glutamine; 50ng/ mL hEGF ; 50 ng/mL bFGF.
Medium D:
   5L DMEM/F12; 5L MCDB-201; 0.1%W/V HAS; 1 µ g/mL linolenic acid; 1 µ g/mL linoleic acid; 0.1% non-essential amino acid; 10-8 mol/L dexamethasone; 10mg/mL ITS; 0.1mmol/L glutamine; 1ng/mL hEGF; 1ng/mL bFGF.
Medium E:
   7L DMEM/F12; 3L MCDB-201; 5%W/V HAS; 100 µ g/mL linolenic acid; 100 µ g/mL linoleic acid ; 5% non-essential amino acid; 10-8 mol/L dexamethasone; 50mg/mL ITS; 10 mmol/L glutamine; 100 ng/mL hEGF; 100ng/mL bFGF.
Medium F:
   6L DMEM/F12; 4L MCDB-201; 2.5%W/V HAS; 50 µ g/mL linolenic acid; 50 µ g/mL linoleic acid ; 2.5% non-essential amino acid; 10-8 mol/L dexamethasone; 25mg/mL ITS; 50 mmol/L glutamine; 50 ng/mL hEGF; 50ng/mL bFGF.

### Example 1:

### Isolation of a population of CD151⁺, CD184⁻, Oct4⁺ pluripotent stem cells by adherence-passage cell culture method

The placentas are delivered to the laboratory in a sterile process. After being minced into 1-5mm³ fragments, the placentas tissues are incubated with 0.5mg/mL collagenase type I for 30 minutes and then 1mg/mL trypsin for 30 minutes at 37°C with gentle agitation for 5 times. The digested mixtures are then passed through a filter to obtain cell suspensions. Next, cells are collected cells by centrifugalization and seeded into 75cm² flasks for culture expansion in medium A for 3-5 days. The cultures are harvested with trypsin and transfer into another 75cm² flasks for further expansion. The adherent cells of passage 6 are harvested and cryopreserved, except some amount of cells being used for quality control.

This population of CD 151 +, CD184-, Oct4+ pluripotent stem cells adheres to the culture flask in 12-72 hours and proliferates rapidly 3-5 days later with the appearance of colonies of fibroblast-like cells first, and then homogenous spindle-like cells with a whirlpool-like array. The primary cultures are harvested with trypsin and transfer to flasks for further expansion. The passage cells can reach 80% confluence in 2-4 days and the amount of 10⁹⁻¹⁰ after 4 weeks. The frequency of colony-forming unit-fibroblast (CFU-F) is about 1 every 400 mononuclear cells.

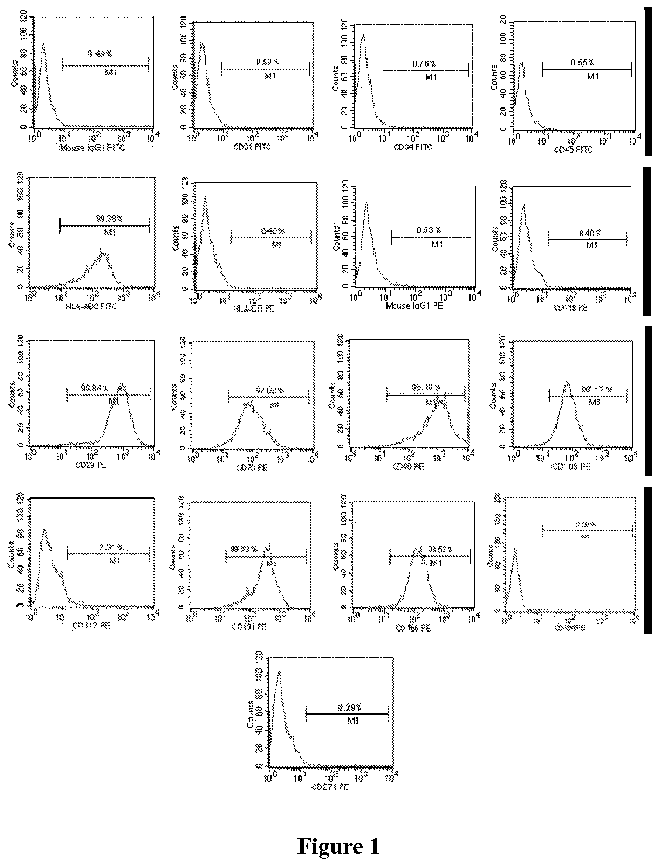

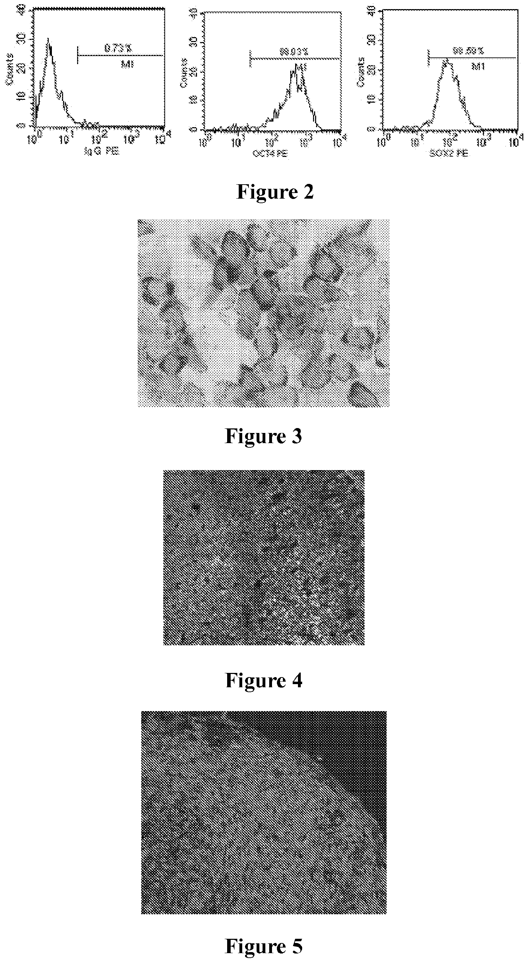

Immunophenotype analysis of passage 6 cells using FACS show that >99% of the pluripotent stem cells population express surface antigen CD151 and do not express CD184, CD34, CD45 and HLA- II (figure 1 and 2). Additionally, these cells express high level of OCT4 and Sox-2 in the cytoplasm.

### Example 2:

### Isolation of a population of CD151⁺, CD184⁻, Oct4⁺ pluripotent stem cells by adherence-passage cell culture method

Same as example 1 except that the medium A is substituted with medium B.

The results are the same as example 1.

### Example 3:

### Isolation of a population of CD151⁺, CD184⁻, Oct4⁺ pluripotent stem cells by adherence-passage cell culture method

Same as example 1 except that the medium A is substituted with medium C.

The results are the same as example 1.

### Example 4:

### Isolation of a population of CD151⁺, CD184⁻, Oct4⁺ pluripotent stem cells by adherence-passage cell culture method

Same as example 1 except that the medium A is substituted with medium D.

The results are the same as example 1.

### Example 5:

### Isolation of a population of CD151⁺, CD184⁻, Oct4⁺ pluripotent stem cells by adherence-passage cell culture method

Same as example 1 except that the medium A is substituted with medium E.

The results are the same as example 1.

### Example 6:

### Isolation of a population of CD151⁺, CD184⁻, Oct4⁺ pluripotent stem cells by adherence-passage cell culture method

Same as example 1 except that the medium A is substituted with medium F.

The results are the same as example 1.

### Example 7:

### Isolation of a population of CD151⁺, CD184⁻, Oct4⁺ pluripotent stem cells by Magnetic Activated Cell Sorting (MACS)

The cords are delivered to the laboratory in a sterile process. After being minced into 1-5mm³ fragments, the cords tissues are incubated with 5mg/mL collagenase type I for 60 minutes and then 5mg/mL a substitute for trypsin (TyrpLE™ Express) for 30 minutes at 37°C with gentle agitation for 6 times. The digested mixtures are then passed through a filter to obtain cell suspensions. Next, according to the common protocol of MACS, CD184⁻ cells are separated from cell suspensions though CD184⁺ magnetic bead firstly, secondly are CD151⁺ CD184⁻ cells though CD151⁺ magnetic bead, and finally are CD151⁺CD184⁻OCT4⁺ cells though OCT4⁺ magnetic bead. Cells are collected cells by centrifugalization and seeded into 75cm² flasks for culture expansion in medium A for 3-5 days. The cultures are harvested with trypsin and transfer into another 75cm² flasks for further expansion. The adherent cells of passage 6 are harvested and cryopreserved, except some amount of cells being used for quality control. Cryopreserve the cells in liquid nitrogen for future use.

### Example 8:

### Characteristics of CD151⁺CD184⁻OCT4⁺ pluripotent stem cells

### 1. High proliferation

Cells, obtained from example 1, are plated at a density of 10⁶ in a 75cm² plastic culture flask. When the well reach 80% confluence 3-4 days later, the culture are transfer into another three flasks for further expansion. After 6-7 passages according to the same passage ratio, the cells amount to 10⁹⁻¹⁰. Moreover, Immunophenotype of these stem cells keep steady in the process of passage (Table 1). Therefore, the preparation of these stem cells can meet the need of large-scale production and clinical use.

**Table 1: Percentages of pluripotent stem cells expressing immunological markers in different passage**

| **Surface marker** | **P6*** | **P20** |
|---|---|---|
| Nestin FITC | 98.67 | 98.73 |
| CD151 PE | 99.22 | 99.96 |
| CD90 FITC | 99.57 | 94.42 |
| HLA-I FITC | 99.79 | 94.45 |
| HLA-II FITC | 2.87 | 0.46 |
| CD34 FITC | 0.51 | 0.05 |
| CD45 FITC | 0.37 | 0.18 |
| CD41 FITC | 0.34 | 0.38 |
| CD29 PE | 99.91 | 99.85 |
| CD 13 PE | 99.73 | 99.54 |
| CD166 PE | 99.52 | 94.34 |
| CD31 PE | 0.82 | 0.05 |
| CD133 PE | 1.23 | 0.21 |
| CD105 PE | 99.78 | 94.7 |
| CD44 PE | 99.95 | 99.79 |
| CD73 PE | 100 | 99.72 |
| CD49e PE | 99.87 | 99.79 |
| CD106 PE | 1.09 | 1.54 |
| CD10 PE | 95.99 | 32.21 |
| CD11b PE | 2.02 | 2.73 |
| CD42b PE | 0.17 | 0.56 |
| GD2 PE | 68.37 | 79.02 |
| CD271 PE | 1.75 | 0.78 |

| **Cytoplasm marker** | **P6** | **P20** |
|---|---|---|
| OCT-4 PE | 98.93 | 96.87 |
| Sox2 PE | 98.59 | 96.36 |
| Nanog FITC | 94.82 | 95.81 |
| c-Myc PE | 99.14 | 98.53 |

| | | |
|---|---|---|
| *P :passage | | |

### 2. Differentiation into three germ layers

### 1) Adipogenic differentiation

The differentiation potential is examined using P6 cells of example 1.

Cells are plated in twenty-four-well plate at a density of 2×10⁴/cm² adding medium A 0.8ml/well. When the well reach 80% confluence, specific induction medium (consist of IMDM, 10% FBS, 1µM dexamethasone, 0.5 mM IBMX, 100 µM indomethacin, 10 µg/ml insulin) substitutes for medium A. The induction medium is changed every 3-4 days for 3 weeks. Then the cells are stained with oil red solution as following steps: a) Rinse the culture with PBS for two times, 5 minutes every time; b) Fix with 4% paraformaldehyde for 15 minutes; c) Rinse with PBS for two times, 5 minutes every time; d) Rinse with 60% isopropyl alcohol; e) add oil red solution and stain for 15 minutes.

Find the results in Figure 3.

Therefore, the pluripotent stem cells of the present invention have the potential of adipogenic differentiation.

### 2) Osteogenic differentiation

The differentiation potential is examined using P6 cells of example 1.

Cells are plated in twenty-four-well plate at a density of 2×10⁴/cm² adding medium A 0.8ml/well. When the well reach 80% confluence, specific induction medium (consist of DMEM-HG, 10% FBS, 10⁻⁸M dexamethasone, 1.5mg/ml b-glycerophosphate, 50µg/ml ascorbic acid) substitutes for medium A. The induction medium is changed every 3-4 days for 3 weeks. Then the cells are stained with von Kossa as following steps: a) Rinse the culture with PBS for two times, 5 minutes every time; b) Fix with 4% paraformaldehyde for 15 minutes; c) Rinse with PBS for two times, 5 minutes every time; d) Add AgNO₃ solution and soak for 10 minutes; e) Treat with ultraviolet light for 10 minutes, rinse with distilled water; f) Soak in 5% sodium sulfite solution, rinse with distilled water.

Find the results in Figure 4.

Therefore, the pluripotent stem cells of the present invention have the potential of osteogenic differentiation.

### 3) Chondrogenic differentiation

The differentiation potential is examined using P6 cells of example 1.

Cell suspensions with a density of 2.5 × 10⁵cells /ml are collected by centrifugation into 15ml centrifuge tube and formed high-density microsphere which are cultured in specific medium consisted of DMEM-HG, 1 × ITS, 1 ×LA-BSA, 100 nM dexamethasone, 50 µg/ml Vitamin C and 10 ng/ml TGF β1 for 21 days. The induction medium is changed every 3-4 days. The cells are embedded in paraffin for sectioning on a microtome into 5 µm slices. Slides are stained with safranine solution.

Find the results in Figure 5.

Therefore, the pluripotent stem cells of the present invention have the potential of chondrogenic differentiation.

### 4) Differentiation into cardiomyocyte

The differentiation potential is examined using P3 cells of example 1.

Cells are plated in six-well plate at a density of 4×10³/cm². When the well reach 80% confluence, specific induction medium (consist of DMEM-HG, 10% FBS, 6µM 5-aza-2'-deoxycytidine) is added. The induction medium is changed into DMEM-HG supplemented with 10% FBS 24 hours later. After 14 days, RT-PCR for cardiomyocyte specific protein markers include α-actin, desmin, MyoD1, myosin and troponin are performed.

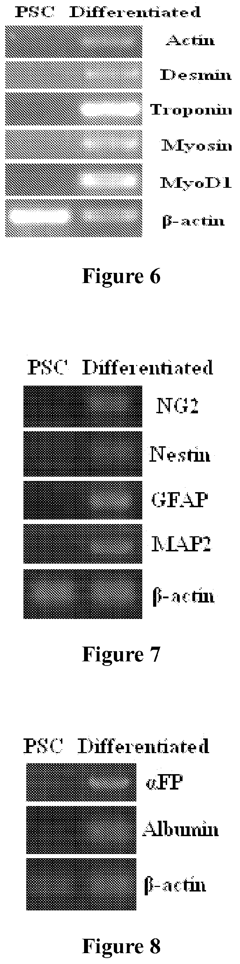

Find the results in Figure 6 and the primers for RT-PCR in Table 2.

Therefore, the pluripotent stem cells of the present invention have the potential of differentiation into cardiomyocyte.

### 5) Neural differentiation

Induction culture is performed as following steps:
Step1: The cells of example 1 are treated with induction medium (consist of DMEM/F12, B27 supplemented with 20 ng/ml EGF, 10 ng/ml bFGF and 10 ng/ml PDGF) for 7 days;
Step2: The cells from step 1 are treated with induction medium (consist of DMEM/F12, B27 supplemented with 10 ng/ml bFGF, 10 ng/ml PDGF 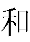 50 ng/ml NGF) for another 7 days. Then, RT-PCR for Nestin, GFAP, MAP2 and NG2 are performed.

Find the results in Figure 7 and the primers for RT-PCR in Table 2.

Therefore, the pluripotent stem cells of the present invention have the potential of neural differentiation.

### 6) Differentiation into hepatocyte

Cells of example 1 are cultured in induction medium consist of IMDM supplemented with 20ng/ml bFGF and 20ng/ml HGF. The medium is changed every 3 days. 14 days later, RT-PCR for AFP and albumin are performed.

Find the results in Figure 8 and the primers for RT-PCR in Table 2.

Therefore, the pluripotent stem cells of the present invention have the potential of differentiation into hepatocyte.

**Table 2: Primers for RT-PCR**

| | **gene** | **primers (5'→3')** | |
|---|---|---|---|
| | Actin | CCTGACCCTGAAGTACCCTATC GCATTTGCGGTGGACGAT | |
| | Desmin | GACCGCTTCGCCAACTACAT TCCTCCAATTCCCGCATCT | |
| | Myosin | CCTGCTGTGCTGTATAACC GTCCATCCCGAAGTCAAT | |
| All above high and into three germ performed pluripotent example 2-7. the some as the | MyoD1 | GCCGCCTGAGCAAAGTAAAT ATAGCGGATGGCGTTGCGCA | experiments of proliferation differentiation layers are with stem cells of The results are example 1. |
| | Troponin | TGGACAAGGTGGATGAAGAG TTCTCGGTGTCCTCCTTCTT | |
| | Nestin | CAGCTGGCGCACCTCAAGATG AGGGAAGTTGGGCTCAGGACTGG | |
| | GFAP | GCACGCAGTATGAGGCAATG CGGTCTTCACCACGATGTT | |
| | MAP2 | CTGGCATTGACCTCCCTAA CTGTTTCCCTTCCCATTTT | |
| | NG2 | CCTTGGCTTTGACCCTGACT CACTGTTGTGGAGCAATACGG | |
| | αFP | AAAAGCCCACTCCAGCATC CAATCCAGCACATCTCCTC | |
| | Albumin | GATTGCCTTTGCTCAGTAT TTGGGTTGTCATCTTTGTG | |
| | β-actin | CTGTCCCTGTATGCCTCTG ATGTCACGCACGATTTCC | |

### Example 9:

### CD151⁺CD184⁻OCT4⁺ pluripotent stem cells being as carrier cells of gene therapy

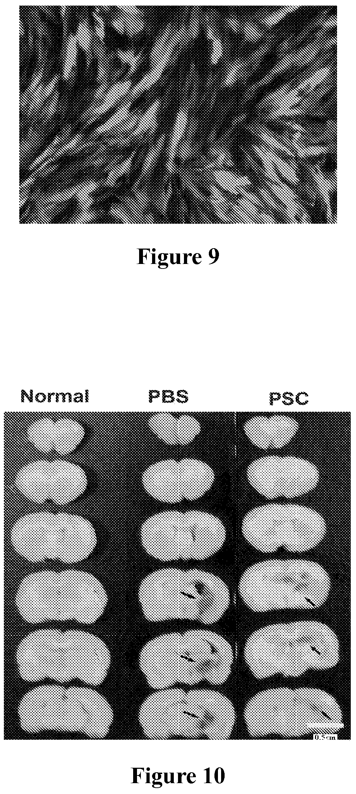

P3-P6 pluripotent stem cells of example 1 are plated in twenty-four-well plate at a density of 1×10⁵ /well. 24 hours later, the medium is then replaced by 1×10⁸PFU /ml Ad-GFP solution diluted with culture medium and the well without virus solution serve as blank control. After 48 hours culture, the cells are identified by fluorescence microscope and transfection efficiency is observed by flow cytometry. The results show that the efficiency is 97.76% 48 hours later transfection of Ad-GFP (Figure 9).

The same experiments are performed with the pluripotent stem cells of example 2-7 and the results are the same as the example 1.

Therefore, the pluripotent stem cells of the present invention can serve as carrier cells of gene therapy.

### Example 10:

### CD151⁺CD184⁻Oct4⁺ pluripotent stem cells for treatment of hemorrhagic brain injured in rats

The study is done with CD151⁺CD184⁻Oct4⁺ pluripotent stem cells of example 1.

The experimental animal models of intracerebral hemorrhage are produced in the base of the method reported by Rosenberg [Rosenberg GA, et al. , Stroke,1990,21:801-807]. Rats were placed in a stereotactic apparatus, and a needle is implanted into the caudate nucleus at coordinates. The rats have 2µl of PBS containing 0.4unit Type VII collagenase infused by a microinfusion pump over 5 minutes. After infusion, the needle was removed and the wound was sutured. The rats recovered from surgery in a warm place with access to food. Behaviors are tested to evaluate the model, including flection and adduction of opposite anterior limb of obstacle side and ipsilateral circling.

24 hours after the models are produced successfully, CD151⁺CD184⁻Oct4⁺ pluripotent stem cells are transplanted into brain through the identical wound. The rats have 10µl of cells suspensions infused by a microinfusion pump over 5 minutes. The control rats have similar infusions of 10µl PBS.

For histology, 28 days after transplantation of the pluripotent stem cells, two groups of experimental rats are killed by the intracardiac injection of 4% paraform. The brains are removed and placed in 4% paraform for 24 hours. Specimens 2mm thick, cut from subependymal region of lateral ventricle, basal ganglia region and hippocampus are taken and observed the extent of the lesion. Find the results in Figure 10. It shows photomicrographs of histologic changes of rat brain 48 hours after intracerebral hemorrhage, in which the arrow indicates the lesion. The figure demonstrates infusion of pluripotent stem cells results in a lesion obviously smaller than that in controls and suggests pluripotent stem cells reduce hemorrhagic brain injured significantly in rats.

The same experiments are done with the pluripotent stem cells of example 2-7 and the results are the same as the example 1.

### Example 11:

### Hematopoiesis-supportive function of CD151+CD184-Oct4+ pluripotent stem cells

P6 CD151⁺CD184⁻Oct4⁺ pluripotent stem cells of example 1 are used for hematopoietic assays.

The cells are plated in twenty-four-well plate at a density of 2.0×10⁴/well and irradiated by ⁶⁰CO with 20Gy. Then, cord blood CD34⁺ cells (2 × 10⁴/well) were seeded on the irradiated layers. The co-cultures are maintained for 8 weeks by weekly replacement of half the medium. Next, cells are harvested and plated in standard methylcellulose culture for colony-forming cell (CFC) assay. The control group is cord blood CD34⁺ cells only.

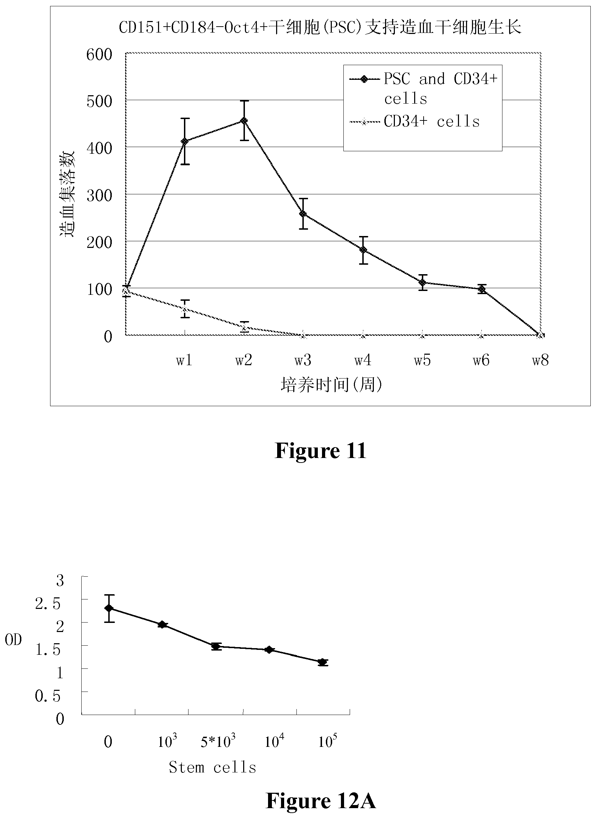

Find the results in Figure 11. Data are presented as mean±SD. The control group forms no CFC after 2 weeks culture, while typical cobblestone areas are observed in CD34⁺ cells/PSC-feeder layer co-cultures after 6 weeks of incubation. These results demonstrate that pluripotent stem cells have hematopoiesis-supportive function for a long time.

The same experiments are done with the pluripotent stem cells of example 2-7 and the results are the same as the example 1.

### Example 12:

### Immunoloregulation of CD151+CD184-Oct4+ pluripotent stem cells

### 1. Immunosuppressive effects of CD151+CD184-Oct4+ pluripotent stem cells investigated by MTT

In this experiment, T cells are co-culture with CD 151+CD184-Oct4+ pluripotent stem cells, the presence of CD151+CD184-Oct4+ pluripotent stem cells inhibit the proliferation of T cells subjected to PHA stimulation (p<0.01) .The PHA mediated stimulation of T cells decrease as the proportion of CD151+CD184-Oct4+ pluripotent stem cells in the culture increase. Inhibition ratio increases from 30.36% to 41.14% and 51.92% when the proportions of 4:1, 2:1 and 1:1 PSCs:T cells in the culture. These results show a dose-dependent inhibitory effect of CD151+CD184-Oct4+ pluripotent stem cells.

### 2. Downmodulation the production of IFN-γ by CD151+CD184-Oct4+ pluripotent stem cells

In this experiment, the secretion of IFN-γ in mice splenocytes subjected to ConA stimulation is significantly inhibited by CD151+CD184-Oct4+ pluripotent stem cells.

Materials and groups are shown in Table 3.

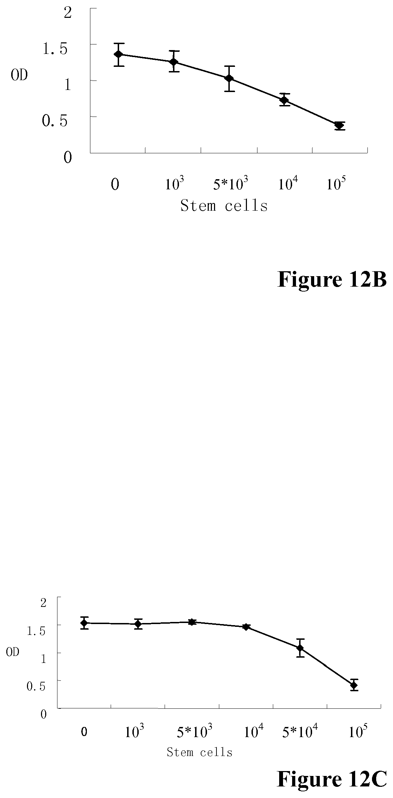

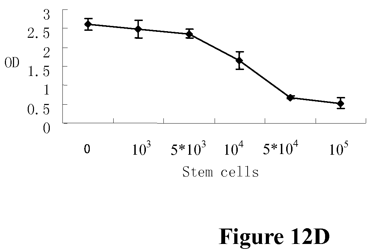

IFN-γ is quantified in the cell culture supernatants by means of ELISA. The results, shown as Figure 12A-12D, demonstrate the significant immunosuppressive effects of CD151+CD184-Oct4+ pluripotent stem cells.

**Table 3: materials and groups**

| | mice splenocyte (/well) | pluripotent stem cells(/well) | ConA |
|---|---|---|---|
| Blank control | - | - | - |
| Negative control | 10⁵ | - | - |
| Positive control | 10⁵ | - | 10ug/ml |
| Experiment group 1 | 10⁵ | 1x10⁵ | 10ug/ml |
| Experiment group 2 | 10⁵ | 5x10⁴ | 10ug/ml |
| Experiment group 3 | 10⁵ | 1x10⁴ | 10ug/ml |
| Experiment group 4 | 10⁵ | 5x10³ | 10ug/ml |
| Experiment group 5 | 10⁵ | 1x10³ | 10ug/ml |

The same experiments are done with the pluripotent stem cells of example 2-7 and the results are the same as the example 1.

### Example 13:

### CD151+CD184-Oct4+ pluripotent stem cells for prevention and treatment of acute graft-versus-host disease (aGVHD)

### 1. Mouse model of aGVHD

A model of bone marrow (BM) transplantation is conducted by transplanting BM cells and splenocytes from C57BL/6(H-2b) mice into BALB/C (H-2d) recipients previously irradiated with dose of 10 Gy. In all instances, 8-week-old male mice are used. Irradiated BALB/C (H-2d) mice are divided into prevention groups and treatment groups. In prevention groups, CD151+CD184-Oct4+ pluripotent stem cells are infused into recipients 5-6 hours before the transplantation of BM cells and splenocytes. And in treatment groups, mice are transplanted BM cells and splenocytes 8-10 hours after irradiation and receive two infusions of CD151+CD184-Oct4+ pluripotent stem cells on day 6 and 12 post-transplantation. The control groups include normal group, irradiation group, model group and homogenous BM cell group. See the details of groups and cell infusion in Table 4. Every animal is observed and recorded clinical situations every day and weight twice a week. The severity of aGVHD is graded. From each animal, samples obtained from liver, lung and skin are collected and stained with H&E and analyzed by a pathologist.

**Table 4: groups for prevention and treatment of aGVHD**

| Group (amount of mouse) | splenocytes C57Bl/6(per mouse) | from BM cells from C57Bl/6 (per mouse) | PSC (per mouse) |
|---|---|---|---|
| Homogenous BM cell group (10) | 0 | 1×10⁷ | - |
| Model group (10) | 1×10⁷ | 1×10⁷ | - |
| Prevention group, low dose (10) | 1×10⁷ | 1×10⁷ | 1×10⁵ |
| Prevention group, high dose (10) | 1×10⁷ | 1×10⁷ | 1×10⁶ |
| Treatment group, low dose (10) | 1×10⁷ | 1×10⁷ | 1×10⁵ |
| Treatment group, middle dose (10) | 1×10⁷ | 1×10⁷ | 5×10⁵ |
| Treatment group, highdose (10) | 1×10⁷ | 1×10⁷ | 1×10⁶ |
| Irradiation group (10) | 0 | 0 | 0 |
| Normal group (10) | 0 | 0 | 0 |

### 2. Results

As expected, most of the animals from irradiation group die within acute stage and the survival rate is 10%. Five animals of Homogenous BM cell group die during observation period and the survival rate is 50%. None of the animals from model group survive within acute stage and the median survival time is 6 days. The survival rates of prevention group (low dose), prevention group (high dose), treatment group (low dose), treatment group (middle dose) and treatment group (high dose) are 20%, 30%, 30%, 50% and 60% respectively, meanwhile the median survival times are 15, 18, 18, 22 and 23 days. Contrast to model group, the infusion of pluripotent stem cells show a significant increase in the survival of transplanted mice (p<0.05, rank sum test) . Moreover, the severity of the aGVHD is diminished when the pluripotent stem cells are infused into recipient mice (p<0.05, U test).

Therefore, infusion of CD151+CD184-Oct4+ pluripotent stem cells prevent the mice from aGVHD and treat the mice suffered from aGVHD.

The same experiments are done with the pluripotent stem cells of example 2-7 and the results are the same as the example 1.

## Claims

1. A method of isolating, purifying and culturally expanding of a population of human pluripotent stem cells from cut human umbilical cord or placenta, the method comprising
(a) cutting the collected human umbilical cord and or placenta tissues by aseptic processing; and
(b) mincing collected tissues into fragments and incubating the fragments with protease and then passing through a filter to obtain primary mononuclear cells; and
(c) selecting CD151⁺, OCT4⁺ and CD184⁻ cells from the mononuclear cells through Magnetic Activated Cell Sorting and cryopreserving in liquid nitrogen.

2. The method of claim 1 wherein said protease is collagenase type I or trypsin.

3. The population of human pluripotent stem cells, isolated by the method of claim 1, which cells are derived from human umbilical cord or placenta and express CD151 and OCT4, and at the same time lack expression of CD 184.

4. The population of pluripotent stem cells of claim 3 for use as carrier cells in gene therapy.

5. The population of pluripotent stem cells of claim 3 for use for treating diseases caused by cell damage or cell aging.

6. The population of stem cells of claim 3 wherein said pluripotent stem cells are used for promoting the in vitro differentiation into lipoblast, osteoblast, chondroblast, myocardial cell, nerve cell, hepatocyte and stimulating the haematogenesis.

7. The population of pluripotent stem cells of claim 3 for use for treating diseases of immunologic abnormality.

8. The population of pluripotent stem cells of claim 3 for use for treating brain damage.

9. The population of pluripotent stem cells of claim 3 for use for treating graft-versus-host disease.

## Patentansprüche

1. Verfahren zum Isolieren, Aufbereiten und kulturellen Erweitern einer Population von menschlichen pluripotenten Stammzellen aus einer geschnittenen menschlichen Nabelschnur oder Plazenta,
wobei das Verfahren folgendes aufweist:
a) Schneiden des gesammelten menschlichen Nabelschnur- und/oder Plazentagewebes durch aseptisches Verarbeiten; und
b) Zerkleinern der gesammelten Gewebe in Fragmente und Inkubatieren der Fragmente mit Protease und dann Hindurchführen durch ein Filter, um primäre mononukleare Zellen zu erhalten; und
c) Auswählen von CD151⁺, OCT4⁺ und CD184⁻ -Zellen aus den mononuklearen Zellen durch magnetisch aktiviertes Zellsortieren und Kryokonservierung in flüssigem Stickstoff.

2. Verfahren nach Anspruch 1, wobei die Protease Kollagenase Typ I oder Trypsin ist.

3. Population von menschlichen pluripotenten Stammzellen, isoliert durch das Verfahren nach Anspruch 1, wobei die Zellen aus einer menschlichen Nabelschnur oder Plazenta erhalten werden und CD151 und OCT4 aufweisen und gleichzeitig CD184 nicht aufweisen.

4. Population von pluripotenten Stammzellen nach Anspruch 3 zur Verwendung als Trägerzelle bei der Gentherapie.

5. Population von pluripotenten Stammzellen nach Anspruch 3 zur Verwendung zur Behandlung von Krankheiten, die durch Zellbeschädigungen oder Zellalterung verursacht werden.

6. Population von Stammzellen nach Anspruch 3, wobei die pluripotenten Stammzellen zum Fördern der Invitro-Differentiation in Liboblast, Osteoblast, Chondroblast, Herzmuskelzellen, Nervenzellen, Hepatocyte und Stimulieren der Hämotogenese verwendet werden.

7. Population von pluripotenten Stammzellen nach Anspruch 3 zur Verwendung zur Behandlung von Krankheiten von immunologischer Abnormalität.

8. Population von pluripotenten Stammzellen nach Anspruch 3 zur Verwendung zur Behandlung von Gehirnschäden.

9. Population von pluripotenten Stammzellen nach Anspruch 3 zur Verwendung zur Behandlung von Transplantal-Wirt-Reaktion.

## Revendications

1. Procédé pour isoler, purifier et étendre par culture une population de cellules souches humaines pluripotentes à partir d'un segment de cordon ombilical ou de placenta, le procédé comportant :
(a) la coupe du cordon ombilical et ou du placenta humain collecté, selon un processus aseptique ; et
(b) le hachage des tissus collectés en fragments et l'incubation de ces fragments avec des protéases et le passage à travers un filtre pour obtenir des cellules primaires mononucléaires ; et
(c) la sélection de cellules CD151⁺, OCT4⁺ et CD184⁻ des cellules mononucléaires au moyen d'un sélecteur de cellules à actionnement magnétique et leur conservation cryogénique dans de l'hydrogène liquide.

2. Procédé selon la revendication 1, dans lequel ladite protéase est du collagène du type I ou de la trypsine.

3. La population de cellules souches humaines pluripotentes isolées selon le procédé de la revendication 1, dont les cellules sont dérivées de cordon ombilical ou de placenta humain, et spécifiquement CD151 et OCT4,
et simultanément ne comportent pas de CD184.

4. La population de cellules souches humaines pluripotentes selon la revendication 3, pour une utilisation en tant que porteur de cellules en thérapie génétique.

5. La population de cellules souches humaines pluripotentes selon la revendication 3, pour une utilisation pour le traitement de maladies causées par des dommages aux cellules ou un vieillissement cellulaire.

6. La population de cellules souches humaines pluripotentes selon la revendication 3, utilisée pour promouvoir la différentiation in vitro en lipoblast, ostéoblast, chondroblast, cellules myocardiques, cellules nerveuses, hépatocyte et pour stimuler l'haématogénèse.

7. La population de cellules souches humaines pluripotentes selon la revendication 3, utilisée pour traiter des maladies liée à une irrégularité immunologique.

8. La population de cellules souches humaines pluripotentes selon la revendication 3, utilisée pour traiter des maladies cérébrales.

9. La population de cellules souches humaines pluripotentes selon la revendication 3, utilisée pour traiter la maladie du greffon contre l'hôte.
